# EUROPEAN PATENT SPECIFICATION

(11) **EP 1 007 142 B1**
(45) Date of publication and mention of the grant of the patent: **24.05.2006**
(21) Application number: 97943534.4
(22) Date of filing: 26.09.1997
(51) Int. Cl.: A61M 37/00, A61B 17/00

(54) **A SPRAYER FOR FIBRIN GLUE**
ZERSTÄUBER FÜR FIBRINKLEBER
PULVERISATEUR POUR COLLE A BASE DE FIBRINE

(30) Priority: 27.09.1996 US 722473
(43) Date of publication of application: 14.06.2000
(73) Proprietor: Thermogenesis Corporation, Rancho Cordova, CA 95670 (US)
(72) Inventor: COELHO, Philip, H., El Dorado Hills, CA 95762 (US); WOLF, Terry, Placerville, CA 95667 (US); MENKE, Peter, Antelope, CA 95843 (US); ALCONE, Jerry, M., Albuquerque, NM 87102 (US)
(74) Representative: Wood, Graham
(86) International application number: PCT/US1997/017116
(87) International publication number: WO 1998/013094

(56) References cited:
- EP-A- 0 634 140
- WO-A-90/01959
- DE-A- 3 408 618
- FR-A- 2 207 728
- US-A- 2 892 457
- US-A- 4 874 368
- US-A- 5 368 563
- US-A- 5 376 079
- US-A- 5 419 491

## Description

### Technical Field

The following invention relates generally to instrumentalities and methodologies for dispensing two discrete components each as a spray, which, when mixed, react and form a biological adhesive. More specifically, the instant invention is directed to a spraying apparatus and methodology for dispensing fibrin glue by the strategic admixture of thrombin from one source and fibrinogen from another source.

### Background Art

Dispensers for fibrin glue should be easy to handle, non clogging, exhibit minimal ullage and precise in their ability to place and meter the fibrin glue at the desired site. To date, all prior art dispensers have been deficient in one or more of the above areas. Because fibrinogen, which is used to produce the fibrin glue is a relatively precious commodity, especially when the fibrinogen is derived autologously, it is imperative that the maximum efficiency possible be experienced when dispensing fibrin glue.

Precise metering has been the bane of devices which dispense fibrin glue. Metering implies that both the precise quantity of fibrin glue will be dispensed and delivered strategically to the appropriate site. Because the glue can obstruct passageways, volume variations which lead to clogging have been a recurring problem.

Impediments that exacerbate the foregoing problems include lack of an ergonomically designed dispensing tool which would facilitate usage in a natural, intuitive way. If the dispensing tool cannot be conveniently grasped, the product being delivered is less likely to be accurately deployed either to the right site or in the right amount.

Another vexing, recurring problem in mixing two component compounds involves the strategic blending of the two components at the appropriate location. A chemical process is associated with mixing the two components. Timing and location are critical. Otherwise, the chemical reaction will likely occur at an unwanted location at an unwanted time. Since fibrin glue is formed substantially immediately upon the contact of thrombin with fibrinogen, and because fibrin glue sets up almost immediately upon its formation, clogging of a dispensing tool can occur. During a surgical procedure, when fibrin glue is used to stem the flow of blood, malfunctions of a fibrin glue delivering tool can have adverse consequences. One corollary to the problem of clogging involves the fact that the thrombin and fibrinogen are typically loaded into the dispensing device with syringes. Should a clog occur, either the dispensing instrumentality has to be cleaned or replaced under aseptic conditions since the fibrinogen and thrombin earmarked for that procedure should be salvaged if at all possible.

Miller, et al 4874368 exemplifies the use of a fibrin glue compounder and sprayer. It requires that initially the user's thumb be fully extended to engage a movable plunger while the index finger and middle finger engage purchase areas on the body of the dispenser. A spray mist requires forceful movement of the thumb towards the index and middle finger. This motion makes it difficult to precisely control the spray site and amount and is ergonomically inefficient.

Document DE 3408618 shows a dispenser with a single syringe and a wobble plate used in the control of dispensation but does not provide for the mixing of two constituents as they are dispensed.

US-A-5 376 079 shows the features of the preamble of claim 1.

### Disclosure of Invention

In a first aspect of the invention there is provided a dispenser for delivering medicament in the form of fibrin glue formed from thrombin and fibrinogen respectively contained in syringes comprising, in combination; a barrel, a dispensing head on said barrel, support means for the first and second syringes on said barrel, including means to depress a plunger in each of the syringes into a cavity of the syringe, each of the syringes, having an outlet coupled to said dispensing head, said dispenser including a hand grip connected to said barrel, a trigger on said hand grip, a rod extending from said depressing means, and a wobble plate mounted to bite with said rod, a pivot lever adapted to cooperate with said wobble plate, said trigger being arranged such that squeezing said trigger pushes against one arm of the pivot lever to pivot the lever and a second arm of the pivot lever moves the wobble plate to cause it to lock to the rod and push the plungers into their respective syringe cavity such that a standardised quanta of fibrinogen and thrombin are dispensed and characterised in that the dispenser includes spray nozzles on said barrel and said spray nozzles are angled toward each other so that fluid is dispelled from the syringes through said nozzles as conical spray mists which communicate while airborne.

Typically the two syringes are in operative communication with a pair of spray nozzles oriented to spray each liquid under the impetus of the trigger. Each spray nozzle sends a fine atomised mist in a diverging conical pattern. The trajectory of one spray overlaps the other approximately 2.65 inches from the tip of the nozzle.

The trigger of the dispensing tool is manipulated by an index finger of the surgeon and the hand hold area (pistol grip) for the device is preferably short to allow clearance so that the dispenser can be used in areas involving tight clearances (e.g. internal body cavities). Unlike prior art devices where the syringes are pushed in a conventional syringe manner (i.e the thumb on the syringe plunger is extended, pushing the plunger towards the index finger and middle finger of the hand which holds the syringe barrel in a typical syringe motion), the structure according to the instant invention lends itself to a more natural; ergonomic motion by the hand so as to provide better control of the spray.

In addition the mechanism incorporating the spray dispensation ensures a uniform dosage will be sprayed upon each manipulation of the spray trigger. This type of standardisation allows a known amount of fibrin glue to be dispensed on each spray actuation.

Because thrombin and fibrinogen are highly reactive, contact between the two components at dispensing areas on prior art devices cause instant clogging. The instant invention includes means to make less likely the instances of clogging, and should clogging occur, remedies to unclog quickly are provided.

One remedy involves the trigger. The trigger mechanism, besides precisely metering out spray, includes structure which allows the pressure exerted on the syringes containing thrombin and fibrinogen to be "backed off" or allowed to creep back to a hydrostatically unstressed position so that it is less likely substance will leak or ooze from the syringes, thereby avoiding a potential source of contamination.

Moreover, the trigger requires a small, natural motion of the surgeon's index finger. The mechanism coupled to the trigger amplifies the trigger motion to produce a predictable, powerful spray with minimal effort, allowing the surgeon control, ease of use and precision.

In addition, a second remedy designs the spray heads as modular and are therefore easily replaceable from the spray head assembly. These factors coupled with a short pistol grip and ergonomic deployment make a user friendly device.

### Industrial Applicability

The industrial applicability of this invention shall be demonstrated through discussion of the following objects of the invention.

Accordingly, it is a primary object of the present invention to provide a new and novel fibrin glue sprayer.

A further object of the present invention is to provide a device as characterized above which is ergonomically sound affording precise control at all times.

A further object of the present invention is to provide a device as characterized above which allows a trigger mechanism to amplify a surgeon's modest movement of the trigger using an index finger, thereby providing a strong spray of predictable characteristics.

A further object of the present invention is to provide a device as characterized above which has a compact profile to allow access of the sprayer into areas normally difficult to access.

A further object of the present invention is to provide a device as characterized above which minimizes the instances of and effects of inadvertent mixing of the reactive components used in the spray device.

A further object of the present invention is to provide a device as characterized above in which finally atomized mists of the two components are caused to focus a precise distance from the nozzles in an overlying concentric, conical pattern for efficient admixture of the two components of the system.

A further object of the present invention is to provide a device as characterized above wherein the components associated with the instant invention lend themselves to mass production techniques and can benefit from manufacturing economies of scale thereby so that should it be economically desirable, the device can be economically disposed after a single use.

A further object of the present invention is to provide a device as characterized above in which the core components of the device are durable and can be readily autoclavable to reprovide aseptic conditions.

Viewed from a first vantage, it is an object of the present invention to provide an: apparatus for dispensing fibrin glue formed from thrombin and fibrinogen, where both the thrombin and the fibrinogen are respectively sequestered in independently operable syringes, the syringes each having a plunger which reciprocate within a hollow syringe and an outlet on the syringe body remote from the plunger, including: means for supporting each of the syringes on a barrel portion of the spray gun, means for constraining plungers of each syringe such that each plunger can reciprocate within a respective hollow of its syringe, said plunger constraining means operatively coupled to a trigger including metering means to carefully control the degree to which the plungers are depressed within the hollows of the syringes to precisely meter the outflow from each syringe, and first and second spray means, one for each syringe, and one said spray means operatively coupled to the outlet of one syringe whereby actuation of the plunger through the trigger dispenses the contents of each said syringe in a fine mist.

Viewed from a second vantage point, it is an object of the present invention to provide a method for forming fibrin glue, the steps including sequestering thrombin in a first syringe, sequestering fibrinogen in a second syringe, orienting each said syringe to be in fluid communication with its own spray head, coupling plungers of each said syringe to a plunger pusher, coupling the plunger pusher to a trigger and metering the fluid exiting each syringe by the trigger.

Viewed from a third vantage point, it is an object of the present invention to provide fibrin glue formed from the coaction of two fine mists: each mist diverges outwardly and each overlaps the other a set distance from its launching source, the fibrin glue formed by a first mist of thrombin projected as an atomized mist in an outwardly diverging pattern from a thrombin source, fibrinogen embodied as a second mist and projected from a fibrinogen source and projected in an outwardly diverging pattern, the first and second outwardly diverging patterns having means to overlap each other during a portion of the respective trajectories remote from sites of spraying so that the thrombin and fibrinogen can mix while aloft and land on a target in substantially overlapping patterns.

These and other objects will be made manifest when considering the following detailed specification when taken in conjunction with the appended drawing figures.

### Brief Description of Drawings

Figure 1 is a perspective view partially exposing an interior portion of the sprayer according to the present invention.
Figure 2 is a an exploded parts perspective of that which is shown in figure 1.
Figure 3 is a perspective view of a tip portion of the device showing first and second syringes docking with the tip.
Figure 4 is a perspective view similar to figure 3 showing the tip in a closed, deployed position.
Figure 5 is a sectional view taken along lines 5-5 of figure 4.
Figure 6 is an exploded detail of a portion of the tip and atomizing spray head shown in figure 5.
Figure 7 is a sectional view along a vertical plane centerline of that which is shown in figure 2.
Figure 8 is a further detail of the tip area showing its ability to hinge from a first position shown in figure 4 to a second position shown in figure 3.
Figure 9 is a detail of the trigger mechanism in a first, at rest position.
Figure 10 is an exaggerated detail of the wobble plate shown in figure 9 in an at rest position.
Figure 11 is a view similar to figure 9 showing the trigger in a deployed position and its effect on the wobble plate.
Figure 12 is an exaggerated view of the effect on the wobble plate in figure 11.
Figure 13 is a view of the trigger in a relaxed position showing the wobble plate returning to the figures 9 and 10 position.
Figure 14 is an exaggerated view of the wobble plate of figure 13 showing its ability to cause "creep back" of the plunger assembly.
Figure 15 is a perspective view of the sprayer being held to be used.
Figure 16 is a perspective view of another embodiment.
Figure 17 is a sectional view of a dispensing head.
Figure 18 is a side view of the figure 17 dispensing head.
Figure 19 is an exploded parts perspective of the figure 17 and 18 dispensing head.
Figure 20 is a front view of the figure 17 dispensing head.
Figure 21 is a perspective view of figure 19 assembled.
Figure 22 is a top plan view of one tube of figure 17.
Figure 23 is a detail of figure 22.
Figure 24 is a plan view of a second tube.

### Best Mode(s) For Carrying Out The Invention

Referring to the drawings, wherein like reference numerals denote like parts throughout the various drawing figures, reference numeral 10 is directed to the spray gun according to the present invention.

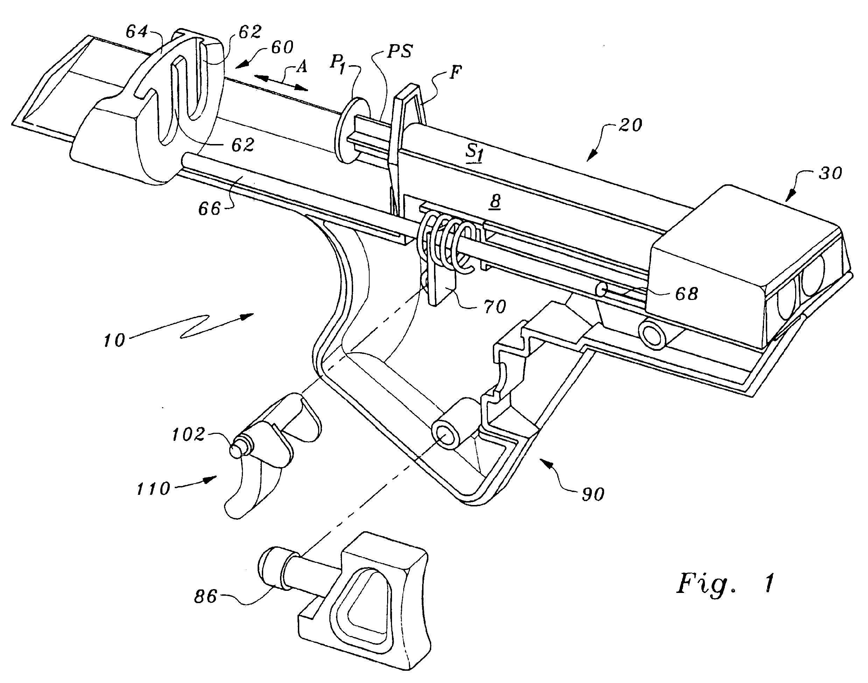

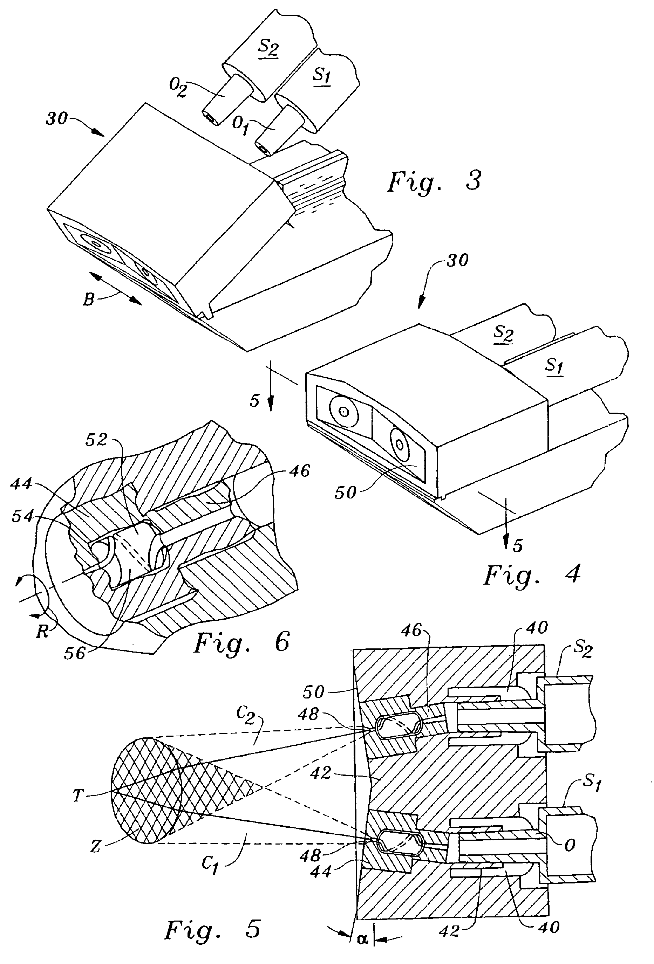

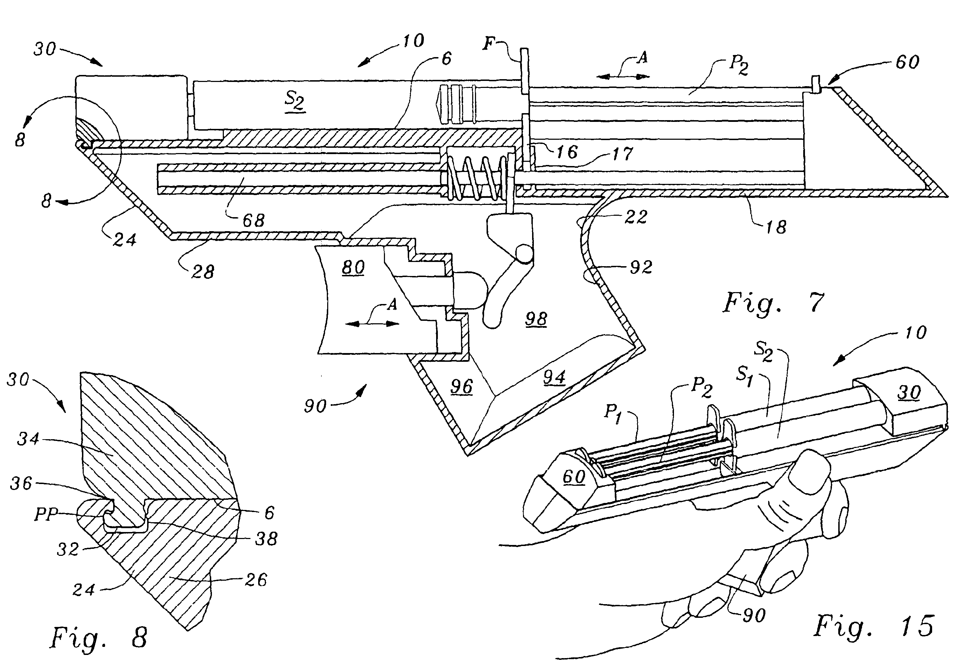

In its essence, and referring to figures 1, 2 and 7, the spray gun 10 includes a barrel area 20 which can accommodate a pair of syringes S1, S2, each of which has plungers P1, P2 which can reciprocate within a hollow of the syringe body between a first contracted position (e.g. figure 1) to a second extended position (e.g. figure 7). The barrel includes, at opposed extremities, a tip area 30 at one end adapted to dock with each outlet of syringes S1, S2 and, at a remote extremity of the barrel 20, a plunger constraining means 60. As the plunger constraining means 60 includes means to engage each of the plungers P1, P2 of the syringes S1, S2, movement of the plunger constraining means and advancing the plunger along the direction of the double ended arrow "A" causes the plunger to reciprocate within the bore of syringes S1, S2. As the plunger pushes material out of syringes S1, S2, it is ejected from the tip 30 in two fine sprays. A combined pistol grip and trigger area 90 is shown in the figures which forms a lower portion of the spray gun 10. Motion of the trigger 80 in the direction of the double ended arrow "A" causes reciprocation through a linkage which amplifies the force applied to the trigger and advances the plunger along the direction of the double ended arrow "A" in a similar manner. As shown in figure 5, once the trigger has been actuated, a finely atomized mist or spray is effortlessly educed from the contents of each of syringes S1, S2 via the outlet tip 30, and a first and second fine mist, outwardly diverging and conical in shape, are provided. The mists are oriented such that each outwardly diverging conical spray converges to allow mixing the sprays and therefore the contents of the two syringes S1, S2. The dispensing surgeon enjoys ergonomic efficiencies and control over a powerfully delivered mist.

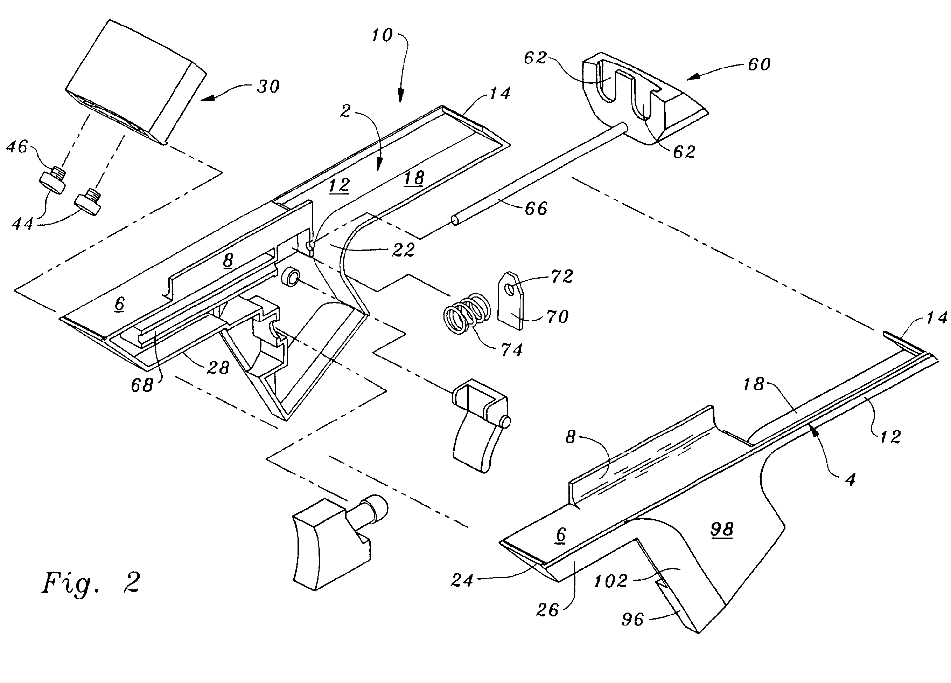

More specifically, and referring to figures 1 and 2, the device 10 is shown as having two major housings, a first housing 2 and a second housing 4. Each housing is substantially the mirror image of the other and therefore minor difference (e.g. male and female frictional fittings) will not be belabored so as not to obscure clarity. The two housings are separable along a vertical plane which runs along the center line of the device.

The barrel portion 20 of each housing includes a planar shelf 6 which underlies the tip 30, an upwardly extending medial wall 8 which separates the first syringe S1 from the second syringe S2 and, at a rearward portion a recessed area including a peripheral wall 12 and an end wall 14. The peripheral wall 12 and the end wall 14 serve as a well within which the plungers P1, P2 of the syringes S1, S2 can be placed with accommodation for the finger grip area F of the syringe.

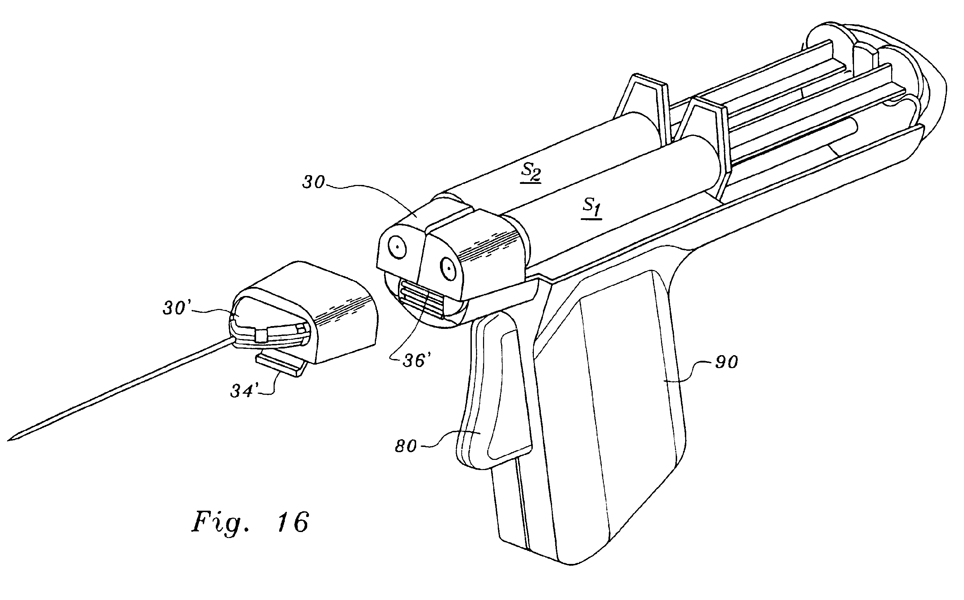

That is, a recess 16 (figure 7) that is formed by a barrier 17 spaced from the front shelf 6 and above a bottom wall 18 of the well secures the syringe in place particularly when the outlets O1, 02 of the syringes S1, S2 are nested within the tip 30 (figure 3) (to be described in greater detail) and a plunger ends P1, P2 of the syringes S1, S2 addresses the plunger constraining means 60 (to be described). The bottom wall 18 transitions to the pistol grip area 90 by means of an arcuate wall 22 (figure 7) having a radius of curvature which provides ergonomic benefit to the bight area of the hand of a user between the thumb and fore finger (figure 15).

The arcuate transition 22 terminates in a pistol grip end wall 92 that receives the topmost portion of the palm of the hand of a user adjacent the bight portion. The pistol grip area terminates in a bottom wall 94 (figure 7) which leads to a front wall 96. The dimension of the front wall 96 is such that it allows the middle finger of the user to rest comfortably thereon with the index finger of the user comfortably residing on the trigger 80. The remaining two fingers of the user fold comfortably under the bottom wall 94 of the pistol grip so that the lessened dimension of the pistol grip 90 allows access to areas of tight clearance.

The pistol grip further includes side walls 98 and a leading edge thereof may have a chamfered contour 102 (figure 2) to provide a smooth transition to an underside of the tip support area shelf 6. More particularly, the shelf 6 has a rearwardly canting leading face 24 which underlies the shelf 6 and communicates with the chamfered contour 102 via a side panel 26. Bottom wall 28 seals the front of the sprayer 10. The front wall 24 and side wall 26 are closed at a bottommost portion thereof with the bottom wall 28. The area above the trigger and below the barrel within the sprayer housing includes a linkage for advancing the plunger and amplifying the effect of motion of the trigger 80 as will be described. It is preferred that each of the housings 2, 4 be formed from moldable material such as plastic and be of durable construction, but can be economically manufactured so that the device can either be disposable or autoclavable.

As shown in figures 3 and 8, the tip 30 can move from a first position (figure 8) to a second position (figure 3) which allows the syringe outlets O1, 02 access to the tip 30. More particularly, the top shelf 6 includes a recess 32 that provides a pivotal and frictional fit with the tip 30. The tip has a substantially J-shaped projection 34 that is complementally received within the recess 32. This defines a pivot point PP where a toe of the J underlies a projection 36 on a leading edge of the shelf 6.

A friction interference fit exists between the tip 30 and a portion of the J remote from the toe and is shown as reference numeral 38. This interference fit 38 between the tip 30 and its underlying support precisely locates the tip when in a deployed (e.g. figure 15) position.

To load the syringes into the spray gun 10, the tip 30 is shown in the upwardly deployed position of figure 3 and the outlets O2, O1 of syringes S2, S1 are disposed within receiving apertures 40 carried in the tip (figure 5). Once the tip 30 is in the figure 4 position, the front portion of the device is operable and ready for use. Figures 3 through 6 show details of the tip geometry.

Each syringes S1, S2 have outlets O1, 02 commonly referred to as a luer coupling. The luer coupling has a conical taper, growing smaller as it extends away from the body of the syringe and is adapted to frictionally reside within a complementally formed opening 40. A sleeve 42 in tangential registry with the outlets O1, 02 receives syringes S1, S2. Thus, the sleeve 42 may have a taper parallel to the taper of the luer coupling so that it diverges outwardly to receive the luer coupling.

Each syringe communicates with its own atomizing nozzle 44. Each atomizing nozzle 44 has a stem 46 (figures 2, 5, 6) having a lesser diameter than the front portion 44 which registers on a front face 50 of the tip block 30. Each of the nozzles 44 are oriented with respect to a center line CL (figure 5) so that the nozzles point towards a common target T approximately 2.56 inches away from the nozzles 44. Thus, each nozzle delivers a substantially conical spray C1, C2 that diverges from a nozzle outlet 48. A zone Z of fibrin glue is thus provided at the target T.

An interior portion of the nozzle body includes atomizer 52 contained within a bore 54 formed within the front portion 44 of the nozzle. The atomizer 52 has an exterior groove 56 causing the atomizer 52 to rotate in the direction of the double ended arrows "R". This helps break up the liquid coming from the syringe to form a fine mist. The grooves 56 on the atomizer 52 allow the atomizer to rotate within the complementally formed recess and provide additional agitation to the mixture coming out.

As shown in figure 5, this structure allows two very fine mists to diverge from the nozzle outlet 48. Having the nozzles 44 canted towards each other allows focused mixing a discrete distance from the nozzles so that when thrombin is used in one syringe and fibrinogen is used in the other syringe, there is a zone Z of overlap that allows good mixing of the two components without either of the components touching the other near a nozzle outlet. This prevents clogging.

Note, however, in the unlikely event that should a clog occur, the tip 30 can be easily removed by overcoming the friction of the J-type hinge of figure 8 by moving the tip 30 along the direction of the double ended arrow "B" shown in figure 3. This allows a new tip 30 to replace an old one. Note also (figure 5) that the front wall 50 of the tip is recessed at its vertical center and subtends an arc defined by an angle α which preferably can be fifteen degrees, but can vary from one to forty-five degrees.

As shown in figure 7, a plunger constraining means 60 is located at an end of the barrel 20 remote from the tip 30. The plunger constraining means includes a pair of upwardly opening U-shaped slots 62 (figures 1 and 2) having a dimension adapted to receive a free end of the plungers P1, P2 normally engaged by a thumb of the user. The thumb engaging area is substantially disc-shaped and slides within the U-shaped slot 62 so that the shaft of the piston PS is on one side of the U-shaped slot and the disc part of the piston is on another. Please see figures 7 and 15.

As shown, clearance is provided between a back wall of the plunger 64 and the U-shaped slots 62 to accommodate the disc part of the plunger. Each syringe is located on opposite sides of the vertical plane of symmetry of the device. The plunger 60 has a relatively large mass and rides within the well at a rear portion of the barrel described hereinabove. The plunger 60 has a contour substantially complemental to the well so that it slides in the well comfortably without binding. This also allows a single syringe to be used in a stable manner without fear of binding because of the support afforded between the walls of the well and the plunger 60.

A bottom portion of the plunger forward of the slots 62 includes a plunger rod 66. One end of the plunger rod 66 is attached to the plunger constraining means or plunger pusher 60 and another end reciprocates within a bore 68 formed from the housings 2, 4 and shown in figures 1, 2 and 7. The bore 68 is formed from arcuate semi-cylindrical sections each formed on inner surfaces of the housing 2, 4 and provide further smooth response to the user when the plunger is being manipulated.

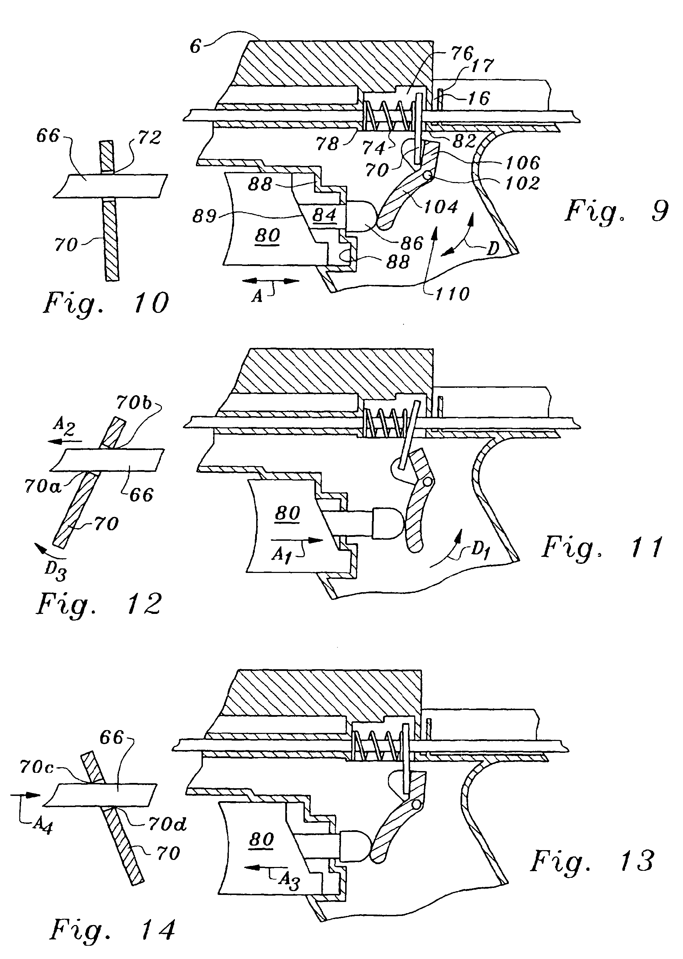

The plunger is activated by the trigger 80. The sequence of steps involving the plunger and trigger can be considered when viewing figures 9 through 14. Briefly, a wobble plate 70 is configured with a hole 72 at an upper portion thereof dimensioned to just pass over the rod 66. The wobble plate 70 is constrained to reside most of the time in a position nearest the bight area 22 of the handle by means of a spring 74 carried within a spring cavity 76 formed on an under side of the barrel shelf 6. The spring 74 is captured on one side by a web 78 formed in the housing. Bore 68 and rod 66 pass through a web aperture. On another end of the spring cavity 76, adjacent the bight area 22, a second apertured web 82 keeps the wobble plate 70 thereagainst by virtue of the spring 74 pressing against a face of the wobble plate 70.

The trigger 80 includes a stem portion 84 that terminates in a bulbous shaped trigger bearing surface 86. The pistol grip portion of the housing adjacent the trigger has an exterior contour complemental to the trigger and includes a limit stop 88 at upper and lower portions to precisely locate an abutment against which the trigger 80 can go no further. This provides assurance to the surgeon that there is no chance of "overshooting", i.e., over dispensing too much fibrin glue as in the prior art. Clearance is provided on the trigger 80 by a canted portion 89 that allows the trigger stem 84 to reciprocate along the direction of the double ended arrow "A".

The bulbous portion of the trigger 86 pushes against pivot lever 110. Pivot lever 110 has a pivot 102 that allows pivotal motion of the lever 110 in the direction of the double ended arrow "D" of figure 9. The lever 110 includes a first leg 104 on one side of the pivot 102 driven by the bulbous end 86 of the trigger 80. A second leg 106, located on pivot lever 110 on an opposite side of pivot 102 contacts a face of the wobble plate 70 remote from the spring 74.

Figure 10 shows the "neutral" angular relationship of the wobble plate 70 to its hole 72 vis-à-vis the plunger rod 66. In figure 11, the trigger 80 is moved in the direction of arrow "A1". This moves pivot lever 110 along D1. In figure 12, wobble plate 70 moves in direction D3 and two points 70a and 70b of the wobble plate 72 bite onto the plunger rod 66 causing motion of the plunger rod in the direction "A2".

Figures 13 and 14 show the trigger 80 being relaxed and allowed to assume an at rest position again and occurs just after having released the trigger of figure 11. The trigger 80 now moves along arrow "A3" of figure 13 by pressure from spring 74. The wobble plate 70 has two other edges 70c and 70d defined by the hole 72 that also bite against the plunger rod 66. This causes slight return of the plunger rod, but only a minute distance A4. The effect of this minute return or "creep back" assures that the plungers P1, P2 on the syringes S1, S2 move back in a similar direction, but only a short distance. This provides a slight negative pressure in the nozzles 44 of the tip 30. This prevents the contents within the syringe from dribbling out, further making nozzle clogging less likely.

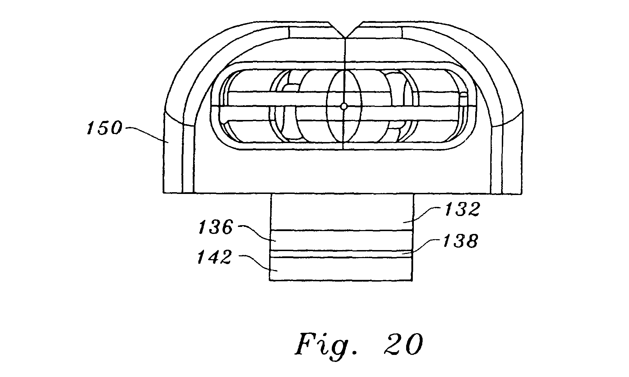

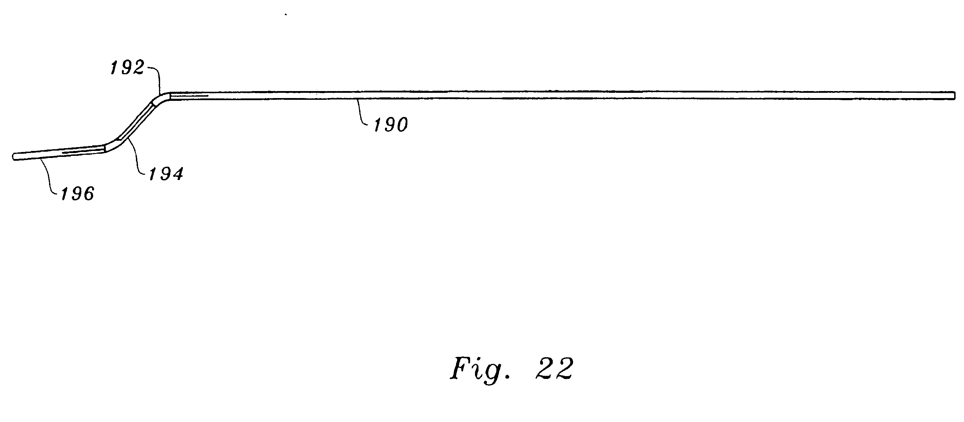

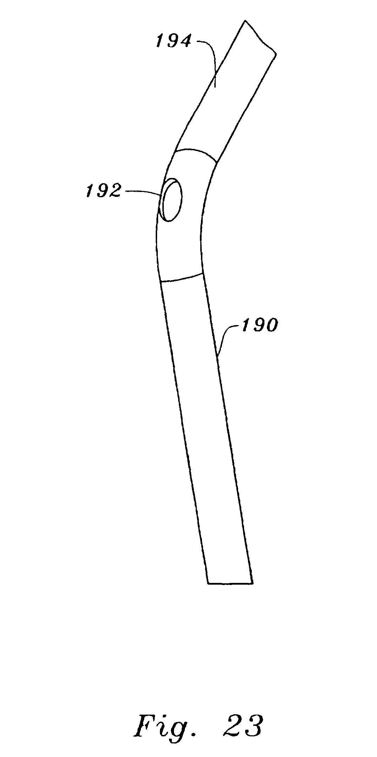

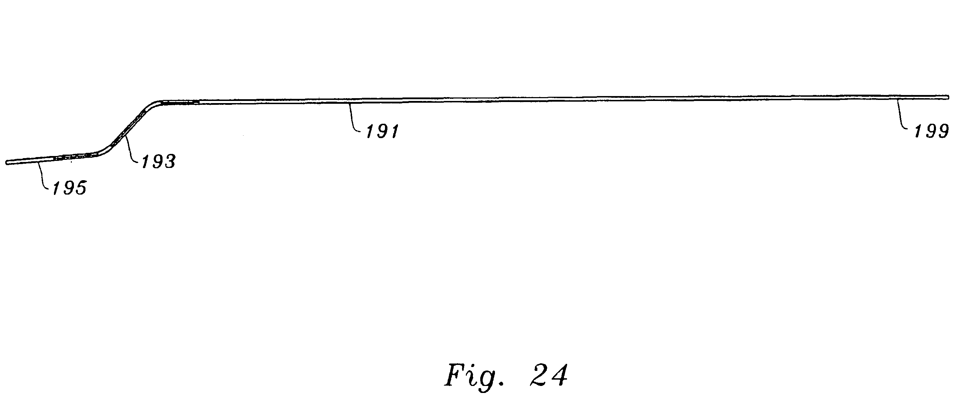

Figures 16 through 24 reflect details of an adapter which converts the sprayer to a dispenser which discharges fibrin glue in a line or dot manner. More specifically, and with reference to figure 16, the tip 30 of the sprayer is replaced with a line-and-dot dispensing tip 30'. In the previous embodiment, the tip 30 moves about an arc permitted by a J-shaped projection 34. The figure 16 embodiment reflects substitution of the J-shaped projection with a locking "C" clip 34'. The clip 34' still rotates, but hereabout a pivot rod 36' to move the tip and provide clearance for the insertion of the two syringes S1, S2, the same as described hereinabove. Thus, the pivot rod 36' replaces the projection 36 of figure 8 on a leading edge of the shelf 6. This arrangement is easier to fabricate and allows removable attachment of either a sprayer having a clip 34' or the line-and-dot tip 30'.

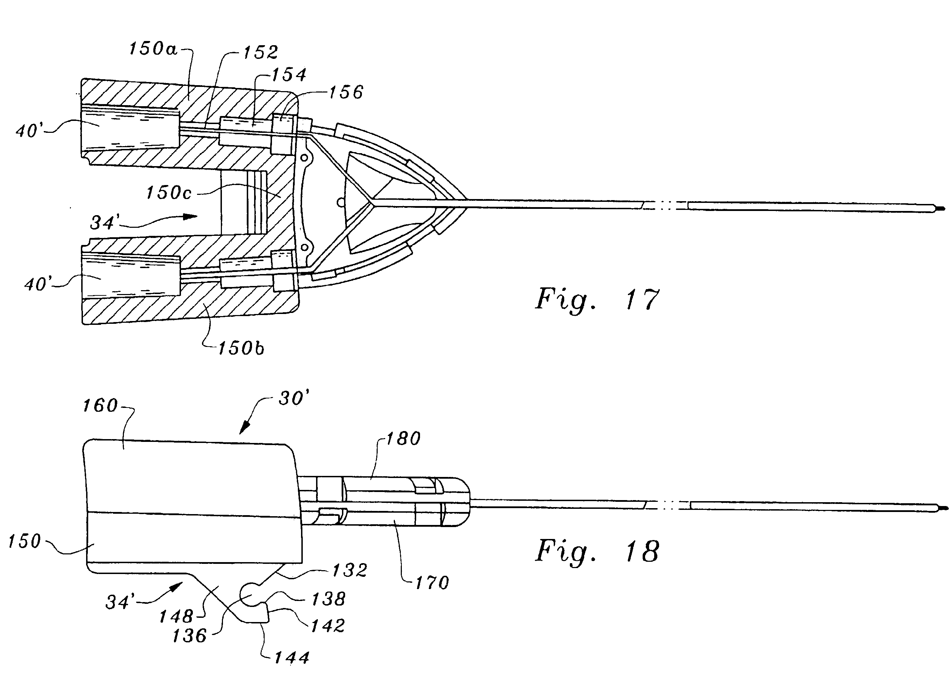

Figure 18 shows details of the complimentarily formed locking clip 34'. A syringe support 150 receives the two syringes. In essence, two skirts 148 extend down from the bottom portions of the syringe support 150. The syringe support 150 (figure 17) includes three major components: a syringe support 150a for syringe S1, a syringe support 150b for syringe S2, and a bridge 150c extending between the syringe supports 150a, 150b at a forwardmost portion thereof. Depending from the bridge 150c and extending rearwardly along facing, adjacent, parallel, inner longitudinal edges of the syringe supports 150a, 150b are the two depending skirts 148 which provide support for the locking "C" clip 34. The locking "C" clip 34 includes an arcuate recess 136 which opens forwardly to receive pivot rod 36'. A first angled face 132 extends upwardly to the syringe support 150 from the arcuate recess 136. A second angled face 138 diverges away from the arcuate recess 136, thereby making the pivot area defined by the arcuate recess 136 easy to locate with the pivot rod 36'. The face 138 communicates with a vertically downwardly extending edge 142 to a horizontal wall 144 which then tapers upwardly and rearwardly, defining a bottom edge of the skirt 148.

Each of the syringe supports 150a, 150b include openings 40' that conically taper downwardly and extend inwardly within the syringe support 150a, 150b. The dimension of the taper allows the luer tip of the syringe to frictionally reside directly therein (i.e., without the need for sleeve 42 of figure 5). The opening 40' communicates with a passageway 152 which opens to a chamber 154 which in turn leads to an outlet 156. The chamber 154 is dimensioned to directly receive the atomizer 52 and the outlet 156 is dimensioned to directly receive the nozzles 44 of the previously discussed embodiment so that the tip 30' can serve the dual purposes of either acting as a line-and-dot dispenser as will be described or as the previously described sprayer.

Figure 18 shows a cowling 160 which is fixed upon the top of the syringe support 150. It has an internal geometry (i.e., opening 40', passageway 152, chamber 154, outlet 156) identical to that which has been described in figure 17 and will not be belabored here. Thus, the figure 17 view shows that the opening, passageway, chamber, outlet, etc. were semi-cylindrical or truncated semi-cones which are completed by their other half on the cowling 160.

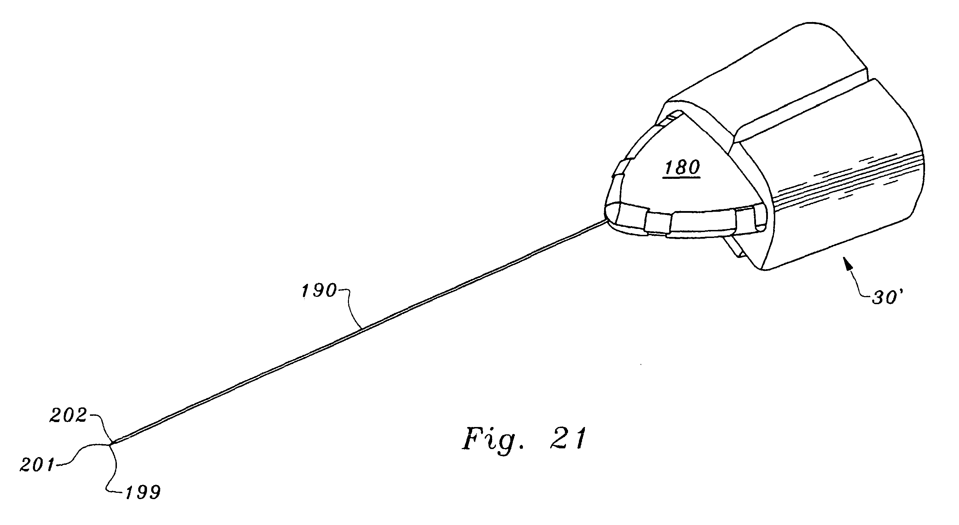

In order to form the line-and-dot dispenser, the problem of having the contents of the two syringes meeting at an appropriate moment is critical so that when they mix and form fibrin glue, they do not clog the output of the line-and-dot dispenser. With respect to figures 21 through 24, the contents of the two syringes S1 and S2 communicate respectively with an inner cylindrical tube 191 and an outer cylindrical tube 190. These tubes 190, 191 are joined together such that the inner cylindrical tube 191 has an end 199 that projects out of the outer cylindrical tube 190 by a short distance (as shown in the drawings, .07 inches). In order to achieve this, the outer cylindrical tube 190 is provided with a hole 192 where the tube 190 bends at a 135° angle from the main body to a minor body portion 194. Thereafter, the tube 190 bends at a 139° included angle to form a free end 196. Thus, the free end 196 is not parallel to the main portion of the tube 190. Once the hole 192 has been placed at the juncture between the main tube body 190 and the first bend 194, the main body of the cylindrical tube 191 is telescoped therewithin. This allows for end 199 to project beyond tube 190. The inner tube 191 also includes an 135° bend transitioning into an angled portion 193 and a free end 195 at 139° so the free end is not parallel with 191.

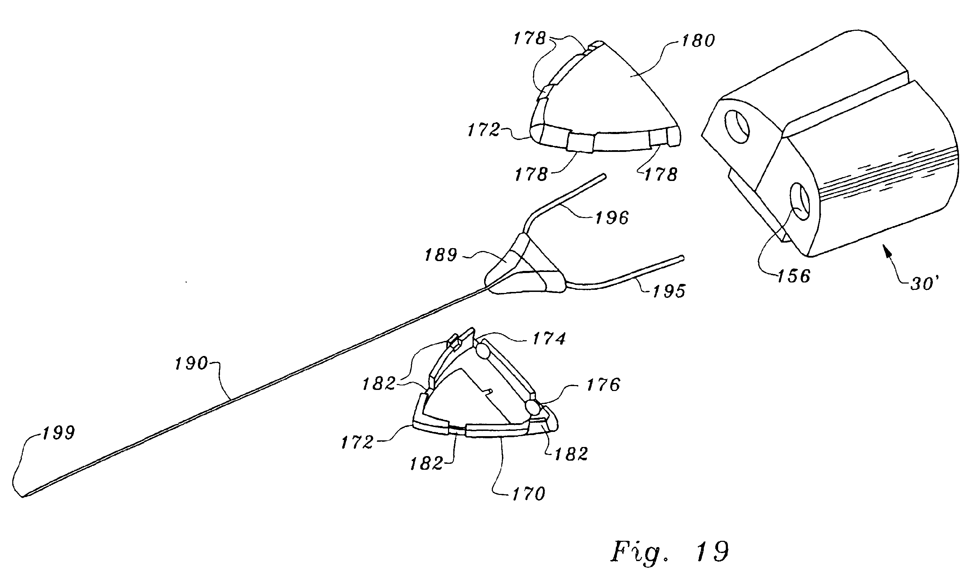

After the inner cylindrical tube 191 has been inserted into the outer cylindrical tube 190, and the free ends 196, 195 are positioned substantially in the same plane, a potting compound 189 (figure 19) is applied at the site of the hole 192. The potting compound 181 and the area of juncture between the two rods are thus sealed from leakage and fixed in position. Thereafter upper and lower substantially triangular shaped shells, 180, 170 respectively, having complemental snap connectors 178, 182 to fix the two shells together. In addition, notches 172, 174, 176 are provided in the shells 180, 170. A leading notch 172 circumscribes the concentric tubes 190, 191 and the two trailing notches 174, 176 respectively engaging the free ends 195, 196. The free ends 195, 196 pass through the nozzle area 156, chamber 154 and passageway 152 and are in alignment with outlets on the luer coupling of the syringes S1, S2. Once the syringes are oriented tightly within the openings 40', and with the luer outlets addressing the tube's free ends 195, 196 in fluid tight engagement, advancement of the plungers as discussed hereinabove cause the contents of the syringes to be expelled from outlet 201 of the inner tube and 202 of the outer tube (figure 21). Since there is an offset 199 between the ends of the tubes 190, 191, when the line-and-dot mechanism is in use (such as one would use a pencil) the contents are mixed without fouling either of the outlets 201, 202.

Having thus described the invention, it should be apparent that numerous structural modifications and adaptations may be resorted to without departing from the scope and fair meaning of the instant invention as described hereinbelow by the claims.

## Claims

1. A dispenser for delivering medicament in the form of fibrin glue formed from thrombin and fibrinogen respectively contained in syringes (S1,S2), comprising, in combination;
a barrel (20)
a dispensing head on said barrel
support means for the first and second syringes (S1,S2) on said barrel including means to depress a plunger (P1,P2) in each of the syringes (S1,S2) into a cavity of the syringe, each of the syringes having an outlet (O1,02) coupled to said dispensing head,
said dispenser including a hand grip (90) connected to said barrel
**characterised in that** said dispenser further comprises
a trigger (80) on said hand grip
a rod (66) extending from said depressing means, and
a wobble plate (70) mounted to bite with said rod,
a pivot lever adapted to cooperate with said wobble plate, said trigger being arranged such that squeezing said trigger pushes against one arm of the pivot lever to pivot the lever and a second arm of the pivot lever moves the wobble plate (70) to cause it to lock to the rod (66) and push the plungers (P1,P2) into their respective syringe cavity such that a standardised quanta of fibrinogen and thrombin are dispensed and wherein
the dispenser includes spray nozzles (44) on said barrel and said spray nozzles are angled toward each other so that fluid is dispelled from the syringes (S1,S2) through said nozzles as conical spray mists which communicate while airborne.

2. The dispenser according to claim 1 **characterised in that** said hand grip further comprises
a first finger rest, said trigger adjacent to and interposed between said barrel and said first finger rest,
a bottom wall adjacent said first finger rest and opposite said trigger and said bottom wall including a second finger rest.

3. The dispenser according to claim 2 **characterised in that** said hand grip has a palm rest adjacent to and interposed between said bottom wall and said barrel, forming an areas at the intersection of said barrel and said palm rest tu accommodate a bight portion of a user's hand between a thumb and index finger.

4. A dispenser according to claim 3 further comprising a trigger housing juxtaposed complementarily about said trigger.

5. A dispenser according to claim 4 **characterised in that** said trigger comprises:
top and bottom sides, said bottom side longer than said top side; and
front and rear sides, said front side concavically formed to better receive an index finger, said rear side complimentarily formed to coact with said trigger housing.

6. A dispenser according to claim 5 **characterised in that** said trigger and said first finger rest surface are substantially parallel to said palm rest.

7. A dispenser according to claim 6 **characterised in that** said trigger and said first finger rest surface are substantially perpendicular to said second finger rest surface.

8. A dispenser according to claim 7 **characterised in that** said trigger and said first finger rest surface are obtusely angled from said barrel.

9. A dispenser according to claim 1 **characterised in that** moving said rod includes incrementally advancing said rod upon successive trigger squeezing and releasing.

10. A dispenser according to claim 1 **characterised in that** said mists converge at a fixed point substantially the same distance from each dispensing head.

11. A dispenser according to claim 1 **characterised in that** said hand grip is provided with an abutment to limit movement of said trigger.

## Patentansprüche

1. Spender für die Ausgabe eines Medikaments in der Form eines Fibrinklebers aus Thrombin und Fibrinogen, die jeweils in Spritzen (S1, S2) enthalten sind, wobei der Spender in Kombination Folgendes umfasst:
einen Zylinder (20),
einen Spenderkopf an dem genannten Zylinder,
Tragmittel für die erste und die zweite Spritze (S1, S2) an dem genannten Zylinder mit Mitteln zum Eindrücken eines Kolbens (P1, P2) in jeder der Spritzen (S1, S2) in einen Hohlraum der Spritze, wobei jede der Spritzen einen mit dem genannten Spenderkopf gekoppelten Auslass (O1, O2) hat,
wobei der genannte Spender einen Handgriff (90) aufweist, der mit dem genannten Zylinder verbunden ist,
**dadurch gekennzeichnet, dass** der genannte Spender ferner Folgendes umfasst:
einen Auslöser (80) an dem genannten Handgriff,
einen Stab (66), der sich von dem genannten Eindrückmittel erstreckt, und
eine Taumelscheibe (70), die so montiert ist, dass sie in den genannten Stab eingreift,
einen Schwenkhebel, der so gestaltet ist, dass er mit der genannten Taumelscheibe zusammenwirkt, wobei der genannte Auslöser so angeordnet ist, dass dieser beim Abdrücken gegen einen Arm des Schwenkhebels gedrückt wird, um den Hebel zu schwenken, und ein zweiter Arm des Schwenkhebels die Taumelscheibe (70) bewegt, um sie zu veranlassen, am Stab (66) anzugreifen und die Kolben (P1, P2) in ihren jeweiligen Spritzenhohlraum zu drücken, so dass eine standardisierte Menge Fibrinogen und Thrombin ausgegeben wird, und wobei der Spender Sprühdüsen (44) an dem genannten Zylinder aufweist und die genannten Sprühdüsen zueinander hin abgewinkelt sind, so dass Fluid aus den Spritzen (S1, S2) durch die genannten Düsen als Sprühnebelkegel ausgestoßen wird, die sich in der Luft miteinander vermischen.

2. Spender nach Anspruch 1, **dadurch gekennzeichnet, dass** der genannte Handgriff ferner Folgendes umfasst:
eine erste Fingeranlage, den genannten Auslöser neben und zwischen dem genannten Zylinder und der genannten ersten Fingeranlage,
eine untere Wand an der genannten Fingeranlage und gegenüber dem genannten Auslöser, wobei die genannte untere Wand eine zweite Fingeranlage aufweist.

3. Spender nach Anspruch 2, **dadurch gekennzeichnet, dass** der genannte Handgriff eine Handflächenanlage neben und zwischen der genannten unteren Wand und dem genannten Zylinder hat, so dass am Schnittpunkt des genannten Zylinders und der genannten Handflächenanlage ein Bereich zur Aufnahme eines Buchtabschnitts zwischen Daumen und Zeigefinger der Hand eines Benutzers entsteht.

4. Spender nach Anspruch 3, der ferner ein Auslösergehäuse umfasst, das sich in komplementärer Nebeneinanderlage um den genannten Auslöser befindet.

5. Spender nach Anspruch 4, **dadurch gekennzeichnet, dass** der Auslöser ferner Folgendes umfasst:
eine Ober- und eine Unterseite, wobei die genannte Unterseite länger ist als die genannte Oberseite; und eine Front- und eine Rückseite, wobei die genannte Frontseite zur besseren Aufnahme eines Zeigefingers konkav geformt ist, wobei die genannte Rückseite komplementär ausgebildet ist, um mit dem genannte Auslösergehäuse zu koagieren.

6. Spender nach Anspruch 5, **dadurch gekennzeichnet, dass** der genannte Auslöser und die genannte erste Fingeranlagefläche im Wesentlichen parallel zu der genannten Handflächenanlage sind.

7. Spender nach Anspruch 6, **dadurch gekennzeichnet, dass** der genannte Auslöser und die genannte erste Fingeranlagefläche im Wesentlichen lotrecht zu der genannten zweiten Fingeranlagefläche sind.

8. Spender nach Anspruch 7, **dadurch gekennzeichnet, dass** der genannte Auslöser und die genannte erste Fingeranlagefläche einen stumpfen Winkel mit dem genannten Zylinder bilden.

9. Spender nach Anspruch 1, **dadurch gekennzeichnet, dass** eine Bewegung des genannten Stabes ein inkrementales Vorwärtsbewegen des genannten Stabes bei aufeinander folgendem Abdrücken und Loslassen des Auslösers beinhaltet.

10. Spender nach Anspruch 1, **dadurch gekennzeichnet, dass** die genannten Nebel an einem festen Punkt im Wesentlichen im selben Abstand von jedem Spenderkopf konvergieren.

11. Spender nach Anspruch 1, **dadurch gekennzeichnet, dass** der genannte Handgriff mit einem Widerlager versehen ist, um die Bewegung des genannten Auslösers zu begrenzen.

## Revendications

1. Pulvérisateur pour délivrer un médicament sous forme d'une colle à base de fibrine, formée à partir d'une thrombine et d'un fibrinogène respectivement contenus dans des seringues (S1, S2) comprenant, en combinaison :
un cylindre (20),
une tête de pulvérisation prévue sur ledit cylindre,
des moyens de support prévus sur ledit cylindre pour les première et seconde seringues (S1, S2), y compris des moyens formant poussoir pour enfoncer un piston (P1, P2) dans chacune des seringues (S1, S2), à l'intérieur d'une cavité de la seringue, chacune des seringues ayant un orifice de sortie (01, 02) couplé à ladite tête de pulvérisation,
ledit pulvérisateur comprenant une poignée (90) connectée audit cylindre, **caractérisé en ce que** ledit pulvérisateur comprend de plus une gâchette (80) prévue sur ladite poignée,
une tige (66) qui se prolonge depuis lesdits moyens formant poussoir et
un disque en nutation (70) monté de sorte à ce que la tige s'engage avec ledit disque en nutation,
un levier pivotant adapté pour coopérer avec ledit disque en nutation, ladite gâchette étant agencée de sorte qu'un appui sur cette gâchette exerce une poussée contre un bras du levier pivotant pour faire pivoter le levier, et un second bras du levier pivotant fait déplacer le disque en nutation (70) pour le bloquer sur la tige (66) et pousser les pistons (P1, P2) à l'intérieur de leur cavité de seringue respective de sorte à faire pulvériser des quanta normalisés de fibrinogène et de thrombine, et dans lequel le pulvérisateur comporte des diffuseurs (44) sur ledit cylindre et lesdits diffuseurs sont inclinés l'un vers l'autre de sorte que le liquide est éjecté hors des seringues (S1, S2) à travers lesdits diffuseurs sous forme de brouillards de pulvérisation coniques qui communiquent lorsqu'ils se trouvent dans l'air.

2. Pulvérisateur selon la revendication 1, **caractérisé en ce que** ladite poignée comprend de plus
un premier appuie-doigt, ladite gâchette étant adjacente audit premier cylindre et audit premier appuie-doigt et interposée entre les deux,
une paroi inférieure adjacente audit appuie-doigt et en face de ladite gâchette, et ladite paroi inférieure comprenant un second appuie-doigt.

3. Pulvérisateur selon la revendication 2, **caractérisé en ce que** ladite poignée a un appuie-paume adjacent à ladite paroi inférieure et audit cylindre et interposée entre les deux, formant une zone à l'intersection dudit cylindre et dudit appuie-paume pour recevoir la partie en forme d'anse comprise entre le pouce et l'index d'un utilisateur.

4. Pulvérisateur selon la revendication 3, qui comprend de plus un logement de gâchette juxtaposé de façon complémentaire autour de ladite gâchette.

5. Pulvérisateur selon la revendication 4, **caractérisé en ce que** ladite gâchette comprend :
des faces supérieure et inférieure, ladite face inférieure étant plus longue que ladite face supérieure ; et des faces antérieure et postérieure, ladite face antérieure étant formée de façon concave pour mieux s'adapter à un index, ladite face postérieure étant formée de manière complémentaire pour agir conjointement avec ledit logement de gâchette.

6. Pulvérisateur selon la revendication 5, **caractérisé en ce que** ladite gâchette et la surface dudit premier appuie-doigt sont sensiblement parallèles audit appuie-paume.

7. Pulvérisateur selon la revendication 6, **caractérisé en ce que** ladite gâchette et la surface dudit premier appuie-doigt sont sensiblement perpendiculaires à la surface dudit second appuie-doigt.

8. Pulvérisateur selon la revendication 7, **caractérisé en ce que** ladite gâchette et la surface dudit premier appuie-doigt sont inclinées selon un angle obtus relativement audit cylindre.

9. Diffuseur selon la revendication 1, **caractérisé en ce que** le mouvement de ladite tige inclut une avance incrémentielle de ladite tige lorsque la gâchette est successivement pressée et relâchée.

10. Diffuseur selon la revendication 1, **caractérisé en ce que** lesdits brouillards convergent vers un point fixe qui se trouve sensiblement à la même distance de chacune des têtes de pulvérisation.

11. Diffuseur selon la revendication 1, **caractérisé en ce que** ladite poignée est pourvue d'une butée qui limite le mouvement de ladite gâchette.
